# EUROPEAN PATENT SPECIFICATION

(11) **EP 1 575 894 B1**
(45) Date of publication and mention of the grant of the patent: **18.03.2009**
(21) Application number: 02781714.7
(22) Date of filing: 23.12.2002
(51) Int. Cl.: C07C 51/285, C07C 63/331

(54) **PROCESS FOR THE PREPARATION OF DIPHENIC ACID**
VERFAHREN ZUR HERSTELLUNG VON DIPHENSÄURE
PROCEDE DE PREPARATION D'ACIDE DIPHENIQUE

(43) Date of publication of application: 21.09.2005
(73) Proprietor: Council of Scientific and Industrial Research, New Delhi 110 001 (IN)
(72) Inventor: TIWARI,Kaushal,K. Central Fuel Research Institute, Dhanbad Jharkhand (IN); BIT, Kumares, C. Central Fuel Research Institute, Dhanbad Jharkhand (IN); THAKUR, Sanjay, K. Central Fuel Research Institute, Dhanbad Jharkhand (IN); MISHRA, Kamlesh,K. Central Fuel Research Institute, Dhanbad Jharkhand (IN); RAO, Sukuru, R. Central Fuel Research Institute, Dhanbad Jharkhand (IN)
(74) Representative: Ahner, Francis
(86) International application number: PCT/IB2002/005612
(87) International publication number: WO 2004/056739

(56) References cited:
- US-A- 3 165 547
- DATABASE WPI Section Ch, Week 198716 Derwent Publications Ltd., London, GB; Class A41, AN 1987-111717 XP002247714 & JP 62 056451 A (KAWASAKI STEEL CORP), 12 March 1987 (1987-03-12)

## Description

### Field of the invention

The present invention relates to a process of synthesis of diphenic acid. More particularly, the present invention relates to a process of synthesis of diphenic acid from phenanthrene. The invention finds its usage in production of high temperature heat resistant resins, engineering plastics, liquid crystalline polymers, pharmaceuticals, agro-chemical industries etc. This method produces an acceptable poly-(amide-imide) resin with adequate thermal stability having good impact resistance, tensile strength or elongation properties and can be drawn as long fibres. Diphenic acid residues act as chain terminators on reaction with the aromatic diamine.

### Background of the invention

Next to naphthalene, phenanthrene is the second largest component of high temperature coal tar. It is concentrated in anthracene oil fraction (300-360°C) of coal tar distillation. It constitutes 30-40% of the crude anthracene cake obtaineed from anthracene oil by cooling and cetrifuging. After recrystalisation of anthracene residue, phenanthrene is recovered from the filtrate by solvent extraction and/or fractional distillation. Phenanthrene and its derivatives, particularly 9:10-Phenanthraquinone, 2,2'-biphenyldicarboxylic acid (Diphenic acid) and 4,4'-biphenyldicarboxylic acid are in growing demand in the world market (annual growth rate 15%) due to their excellent performance in many newly developed applications, such as production of high temperature heat resistant resins, engineering plastics, liquid crystalline polymers, pharmaceuticals and agro-chemical industries etc.

Reference is made to US Patent 4,352,922 wherein basic chemistry of phenanthrene-derived poly-(amide-imide) resins is described. Although this method produces an acceptable poly-(amide-imide) resin with adequate thermal stability, the resin does not have very good impact resistance, tensile strength or elongation properties and cannot be drawn as long fibres. It is thought that the deficiencies in the physical properties of the resin are due to the low molecular weight of the phenanthrene / formaldehyde reaction product and the presence therein of many oligomers of phenanthrene having terminal moieties linked to the chain at either the 9 or 10 position. On oxidation, such a reaction product will give rise to diphenic acid residues which will act as chain terminators on reaction with the aromatic diamine. R. Behrend, Zeit. Phys. Chem., 1892, 9, p. 405; 10, p. 265 describes oxidation of phenanthrene by reacting alcoholic solution of phenanthrene with chromic acid, first to phenanthre-4uinone, and then to diphenic acid but the yield and purity are poor.

Reference made to US Patent 4,373,089 wherein phenanthrene is converted to its 9, 10 diol derivative via 9, 10 phenanthraquinone. The phenanthrene is oxidised by a mild oxidising agent, such as potassium dichromate, to produce the phenanthraquinone which is reduced to the 9, 10 phenanthrene diol by a mild reducing agent, such as sulphur dioxide. Sulphur dioxide is a convenient reducing agent because it is possible to bubble it through a solution of the phenanthraquinone to produce the 9, 10, diol derivative. The derivative can be protected from reoxidation by a blanket of an inert gas such as nitrogen. According to a second aspect of the present invention, there is provided a polyimide resin comprising the condensation product of a reaction between an aromatic diamine and a polycarboxylated product formed by reacting the 9, 10-diol derivative of a phenanthrene with formaldehyde and oxidising the reaction product to produce keto groups bridging the diphenic acid moieties produced. According to a third aspect of the present invention, there are provided intermediates in the formation of a polyimide resin comprising firstly the reaction product of a 9, 10-diol derivative of a phenanthrene with formaldehyde in the presence of an acid catalyst, and secondly the reaction product which has been oxidised to produce keto groups bridging the diphenic acid moieties produced.

US 3 165 547 teaches a process for producing diphenic acid wherein phenanthrene and concentrated acetic acid are heating in a solvent like benzene or nitrobenzene. This document does not suggest to carry out the reaction without the use of a solvent. Furthermore, hydrogen peroxide is not added drop-wise.

Prior art search for production of diphenic acid was done based on literature survey and patent databases and did not yield any relevant references.

### Objects of the invention

The main object of the invention is to provide a process of synthesis of diphenic acid from phenanthrene which obviates the drawbacks as detailed above.

Another object of the invention is to obtain 99% pure diphenic acid.

Yet another object of the invention is to provide a process for the preparation of diphenic acid which is simple and eco-friendly.

It is another object of the invention to provide a process for the synthesis of diphenic acid which is economical.

### Summary of the invention

Accordingly the present invention provides a process for the preparation of diphenic acid from phenanthrene which comprises
(i) heating phenanthrene and glacial acetic acid,
(ii) adding drop-wise a pre-determined amount of 30% hydrogen peroxide,
(iii) heating the resulting mixture after completion of drop wise addition of hydrogen peroxide,
(iv) distilling the resulting mixture under reduced pressure to make the volume half,
(v) cooling the mixture till diphenic acid crystalises out,
(vi) filtering the cooled mixture and boiling the residue after adding 10% solution of sodium carbonate and activated charcoal,
(vii) filtering and discarding the residue;
(viii) acidifying the filtrate with hydrogen chloride;
(ix) cooling the resultant mixture till more diphenic acid crystallises out;
(x) repeating filtration till pure diphenic acid is obtained.

In one embodiment of the invention, the heating in step (i) above is done in a reactor at a temperature in the range of 75 to 85°C.

In another embodiment of the invention, 30% hydrogen peroxide is added drop wise in an amount in the range of 100 to 300 ml, for a time period in the range of 30 to 60 minutes.

In yet another embodiment of the invention, the heating in step (iii) is done for a time period in the range of 3 to 7 hours.

In yet another embodiment of the invention, the residue in step (vi) is boiled at 100°C after adding 10% solution of sodium carbonate and activated charcoal for decolouration.

In a further embodiment of the invention, the acid is added to maintain the pH of the mixture in the range of 3 to 4.5.

In another embodiment of the invention, the amounts of phenanthrene and glacial acetic acid added are in the ratio of 1: 10 to 1:12 (w/w).

In a further embodiment of the invention, the purity of diphenic acid produced is 99%.

### Detailed description of the invention

Phenanthrene and glacial acetic acid are added together in a reactor and heated up within 75 - 85°C. To the resulting mixture, 100-300 ml of 30% hydrogen peroxide solution is added drop wise, which takes between 30-60 minutes. After completion of addition of hydrogen peroxide solution, the temperature of 75-85°C is further maintained for a time period ranging between 3 to 7 hours. The resulting mixture is subjected to distillation, under reduced pressure, to make the volume half and the mass is allowed to cooL On cooling, considerable amount of diphenic acid crystalises out.

The cooled mixture is filtered and the residue is boiled at 100°C after addition of 10% solution of sodium carbonate and activated charcoal (for decolouration) and subjected to filtration after which the residue is discarded and the filtrate is acidified with hydrogen chloride to maintain the pH at 4.5 and cooled where diphenic acid crystallises out. This process is repeated several times till pure diphenic acid is obtained having melting point at 228 - 229°C.

The phenanthrene and glacial acetic acid added are preferably in a ratio of 1: 10 (w/w). It is observed that the purity of diphenic acid produced is about 99%.

The novelty of the present invention resides in slow and controlled oxidation of phenanthrene (drop wise addition of the oxidising agent at a specific temperature range and a specific time range, which was unknown in the prior art). This method produces an acceptable poly-(amide-imide) resin with adequate thermal stability having good impact resistance, tensile strength or elongation properties and which can be drawn as long fibres. Diphenic acid residues act as chain terminators on reaction with the aromatic diamine. The method of the invention can be used in many newly developed field of applications e.g. production of heat resistant resins, engineering plastics, liquid crystalline polymers, pharmaceuticals, agro-chemical industries etc. from phenanthrene, which was otherwise unutilised due to cost factors. The present process was proved to enhance the yield of diphenic acid drastically.

The following examples are given by way of illustration and should not be construed to limit the scope of the present invention.

### Example-1

25 grams of Phenanthrene and 253 grams glacial acetic acid are added together in a reactor and heated up to 85°C. To the resulting mixture, 100 ml of 30% hydrogen peroxide solution is added drop wise, which normally takes 40 minutes. After completion of addition of hydrogen peroxide solution, the temperature of 85°C is further maintained for a time period of 6 hours. The resulting mixture is subjected to distillation, under reduced pressure, to make the volume half and the mass is allowed to cool. On cooling, considerable amount of diphenic acid crystalises out. The cooled mixture is filtered and the residue is boiled at 100°C after addition of 10% solution of sodium carbonate and activated charcoal (for decolouration) and subjected to filtration after which the residue is discarded and the filtrate is acidified with hydrogen chloride to maintain the pH at 4.5 and cooled where diphenic acid crystallises out. This process is repeated several times till pure diphenic acid is obtained having melting point at 228°C. Yield obtained was 11 grams.

### Example-2

25 grams Phenanthrene and 253 grams glacial acetic acid are added together in a reactor and heated up to 85°C. To the resulting mixture, 200 ml of 30% hydrogen peroxide solution is added drop wise, which normally takes 40 minutes. After completion of addition of hydrogen peroxide solution, the temperature of 85°C is further maintained for a time period 6 hours. The resulting mixture is subjected to distillation, under reduced pressure, to make the volume half and the mass is allowed to cool. On cooling, considerable amount of diphenic acid crystalises out. The cooled mixture is filtered and the residue is boiled at 100°C after addition of 10% solution of sodium carbonate and activated charcoal (for decolouration) and subjected to filtration after which the residue is discarded and the filtrate is acidified with hydrogen chloride to maintain the pH at 4.5 and cooled where diphenic acid crystallises out. This process is repeated several times till pure diphenic acid is obtained having melting point at 229°C. The yield obtained was 17 grams.

### Example-3

25 grams Phenanthrene and 253 grams of glacial acetic acid are added together in a reactor and heated up to 85°C. To the resulting mixture, 88 ml of 30% hydrogen peroxide solution is added drop wise, which normally takes 30 minutes. After completion of addition of hydrogen peroxide solution, the temperature of 80°C is further maintained for a time period of 3.5 hours. The resulting mixture is subjected to distillation, under reduced pressure, to make the volume half and the mass is allowed to cool. On cooling, considerable amount of diphenic acid crystalises out. The cooled mixture is filtered and the residue is boiled at 100°C after addition of 10% solution of sodium carbonate and activated charcoal (for decolouration) and subjected to filtration after which the residue is discarded and the filtrate is acidified with hydrogen chloride to maintain the pH at 4.5 and cooled where diphenic acid crystallises out. This process is repeated several times till pure diphenic acid is obtained having melting point at 229°C. The yield is 12 gms.

**The main advantages of the present invention are:**
1. The process is very simple and eco-friendly.
2. The yield of the product is very high in comparison to prior art.
3. No side reactions are involved in the process.

## Claims

1. A process for the preparation of diphenic acid from phenanthrene which comprises
(i) heating phenanthrene and glacial acetic acid at a temperature in a range of 75 to 85°C,
(ii) adding drop-wise a pre-determined amount of 30% hydrogen peroxide, at a rate corresponding to 100 to 300 ml over a period of from 30 to 60 minutes,
(iii) heating the resulting mixture for 3 to 7 hours after completion of drop-wise addition of hydrogen peroxide,
(iv) distilling the resulting mixture under reduced pressure to make the volume half,
(v) cooling the mixture until diphenic acid crystallizes out,
(vi) filtering the cooled mixture, and boiling the residue after adding 10% solution of sodium carbonate and activated charcoal,
(vii) filtering and discarding the residue,
(viii) acidifying the filtrate with hydrogen chloride,
(ix) cooling the resultant mixture until more diphenic acid crystallizes out, and
(x) repeating filtration until pure diphenic acid is obtained.

2. A process as claimed in claim 1 wherein the residue in step (vi) is boiled at 100°C after adding 10% solution carbonate and activated charcoal for discoloration.

3. A process as claimed in claim 1 wherein the acidifying in step (viii) comprises adding the hydrogen chloride in an amount sufficient to maintain pH of the mixture in the range of 3 to 4.5.

4. A process as claimed in claim 1 wherein the amounts of phenanthrene and glacial acetic acid are in ratio of 1:10 to 1:12 (w/w).

5. A process as claimed in claim 1 wherein the purity of diphenic acid produced is 99%.

6. A process for the preparation diphenic acid from phenanthrene according to claim 1 which comprises heating phenanthrene and glacial acetic acid in a reactor at a temperature in the range of 75 to 85°C, adding 30% hydrogen peroxide drop-wise, the amount of hydrogen peroxide being in the range of 100 to 300 ml, for a time period in the range of 30 to 60 minutes, heating the resulting mixture after completion of drop-wise addition of hydrogen peroxide for a time period in the range of 3 to 7 hours, subjecting the resulting mixture to distillation, under reduced pressure to make the volume half, cooling the mixture until a considerable amount of diphenic acid crystallizes out, filtering the cooled mixture; boiling the residue at 100°C after addition of 10% solution of sodium carbonate and activated charcoal for discoloration, filtering and discarding; acidifying the filtrate with hydrogen chloride to maintain the pH at 4.5; cooling the resultant mixture until diphenic acid crystallizes out; and repeating filtration several times until pure diphenic acid is obtained having a melting point at 228-229°C.

## Patentansprüche

1. Verfahren zur Herstellung von Diphensäure mit Phenanthren, das Folgendes umfasst:
(i) das Erwärmen von Phenanthren und Eisessig mit einer Temperatur im Bereich von 75 bis 85 °C,
(ii) das tropfenweise Hinzufügen eines vorbestimmten Betrages von 30% Wasserstoffperoxid mit einer Menge von 100 bis 300 ml über eine Periode von 30 bis 60 Minuten,
(iii) das Erwärmen der sich ergebenden Mischung über 3 bis 7 Stunden nach Vollendung des tropfenweises Hinzufügen von Wasserstoffperoxid,
(iv) das Destillieren der sich ergebenden Mischung unter reduziertem Druck zur Halbierung des Volumens,
(v) das Kühlen der Mischung bis sich Diphensäure herauskristallisiert,
(vi) die Filterung der gekühlten Mischung und das Sieden des Rückstands nach Hinzufügen von 10% Lösung Natriumcarbonat und Aktivkohle,
(vii) die Filterung und das Aussondern des Rückstands;
(viii) das Ansäuern des Filtrats mit Hydrogenchlorid;
(ix) das Kühlen der sich ergebenden Mischung bis sich mehr Diphensäure herauskristallisiert;
(x) die wiederholte Filterung bis reine Diphensäure erhalten wird.

2. Verfahren nach Anspruch 1, worin der Rückstand in Schritt (vi) nach Hinzufügen von 10% Lösung Natriumcarbonat und Aktivkohle zur Entfärbung bei 100 °C gesiedet wird.

3. Verfahren nach Anspruch 1, worin das Ansäuern in Schritt (viii) das Hinzufügen von Hydrogenchlorid in einer Menge, die ausreicht, um den pH-Wert der Mischung im Bereich von 3 bis 4,5 aufrecht zu erhalten, umfasst.

4. Verfahren nach Anspruch 1, worin die Mengen von Phenanthren und Eisessig im Verhältnis von 1 : 10 bis 1 : 12 (Gew./Gew.) sind.

5. Verfahren nach Anspruch 1, worin sich die Reinheit der erzeugten Diphensäure auf 99% beläuft.

6. Verfahren zur Herstellung von Diphensäure mit Phenanthren nach Anspruch 1, umfassend das Erwärmen von Phenanthren und Eisessig in einem Reaktor bei einer Temperatur im Bereich von 75 bis 85 °C, das tropfenweise Hinzufügen von 30% Wasserstoffperoxid, wobei die Menge von Wasserstoffperoxid im Bereich von 100 bis 300 ml gehalten wird, für einen Zeitabschnitt im Bereich von 30 bis 60 Minuten, das Erwärmen der sich ergebenden Mischung nach Vollendung des tropfenweisen Hinzufügens von Wasserstoffperoxid für einen Zeitabschnitt im Bereich von 3 bis 7 Stunden, das Unterziehen der sich ergebenden Mischung einer Destillation, unter reduziertem Druck zur Halbierung des Volumens, das Kühlen der Mischung bis sich eine bemerkenswerte Menge Diphensäure herauskristallisiert, die Filterung der gekühlten Mischung; das Sieden des Rückstandes bei 100 °C nach Hinzufügung von 10% Lösung Natriumcarbonat und Aktivkohle zur Entfärbung, die Filterung und das Aussondern; das Ansäuern des Filtrats mit Hydrogenchlorid, um den pH-Wert bei 4,5 aufrecht zu erhalten; das Kühlen der sich ergebenden Mischung bis sich Diphensäure herauskristallisierte; die mehrmalige wiederholte Filterung bis reine Diphensäure mit einem Schmelzpunkt bei 228 bis 229 °C erhalten wird.

## Revendications

1. Procédé de préparation d'acide diphénique à partir de phénanthrène, qui comprend
(i) le chauffage de phénanthrène et d'acide acétique glacial à une température comprise dans la plage allant de 75 à 85 °C,
(ii) l'ajout goutte à goutte d'une quantité prédéterminée de peroxyde d'hydrogène à 30 %, à un taux correspondant de 100 à 300 ml sur une période de 30 à 60 minutes,
(iii) le chauffage du mélange résultant pendant 3 à 7 heures après l'achèvement de l'ajout goutte à goutte de peroxyde d'hydrogène,
(iv) la distillation du mélange résultant sous pression réduite pour réduire de moitié le volume,
(v) le refroidissement du mélange jusqu'à la cristallisation de l'acide diphénique,
(vi) la filtration du mélange refroidi, et l'ébullition du résidu après l'ajout d'une solution de 10 % de carbonate de sodium et de charbon actif,
(vii) la filtration et l'élimination du résidu,
(viii) l'acidification du filtrat avec du chlorure d'hydrogène,
(ix) le refroidissement du mélange résultant jusqu'à ce que plus d'acide diphénique cristallise, et
(x) la répétition de la filtration jusqu'à l'obtention d'acide diphénique pur.

2. Procédé selon la revendication 1, dans lequel le résidu de l'étape (vi) est porté à ébullition à 100 °C après l'ajout d'une solution de 10 % de carbonate de sodium et de charbon actif pour la décoloration.

3. Procédé selon la revendication 1, dans lequel l'acidification de l'étape (viii) comprend l'ajout du chlorure d'hydrogène en une quantité suffisante pour maintenir le pH du mélange dans la plage de 3 à 4,5.

4. Procédé selon la revendication 1, dans lequel les quantités de phénanthrène et d'acide acétique glacial sont dans un rapport de 1/10 à 1/12 (m/m).

5. Procédé selon la revendication 1, dans lequel la pureté de l'acide diphénique produit est de 99 %.

6. Procédé de préparation d'acide diphénique à partir de phénanthrène selon la revendication 1, qui comprend le chauffage de phénanthrène et d'acide acétique glacial dans un réacteur à une température comprise dans la plage allant de 75 à 85 °C ; l'ajout goutte à goutte de peroxyde d'hydrogène à 30 %, la quantité de peroxyde d'hydrogène étant comprise dans la plage allant de 100 à 300 ml, sur une période de temps comprise dans la plage allant de 30 à 60 minutes ; le chauffage du mélange résultant après l'achèvement de l'ajout goutte à goutte de peroxyde d'hydrogène pendant une période de temps comprise dans la plage allant de 3 à 7 heures ; la soumission du mélange résultant à une distillation, sous pression réduite pour réduire de moitié le volume ; le refroidissement du mélange jusqu'à la cristallisation d'une quantité considérable d'acide diphénique, la filtration du mélange refroidi ; l'ébullition du résidu à 100 °C après l'ajout d'une solution de 10 % de carbonate de sodium et de charbon actif pour la décoloration, sa filtration et son élimination ; l'acidification du filtrat avec du chlorure d'hydrogène pour maintenir le pH à 4,5 ; le refroidissement du mélange résultant jusqu'à la cristallisation d'acide diphénique ; et la répétition de la filtration plusieurs fois jusqu'à l'obtention d'acide diphénique pur ayant un point de fusion à 228-229° C.
